# EUROPEAN PATENT SPECIFICATION

(11) **EP 2 900 208 B1**
(45) Date of publication and mention of the grant of the patent: **14.03.2018**
(21) Application number: 13771031.5
(22) Date of filing: 20.09.2013
(51) Int. Cl.: A61K 47/32, A61K 9/70, A61K 8/36, A61K 8/81, A61Q 19/00

(54) **PEELABLE FILM-FORMING COMPOSITIONS**
ABZIEHBARE FILMBILDENDEN ZUSAMMENSETZUNGEN
COMPOSITIONS FILMOGÈNES PELABLES

(30) Priority: 28.09.2012 US 201213631144
(43) Date of publication of application: 05.08.2015
(73) Proprietor: Johnson & Johnson Consumer Inc., Skillman, NJ 08558 (US)
(72) Inventor: BAHADUR, Prashant, North Brunswick, NJ 08902 (US); WU, Jeffrey M., Princeton, New Jersey 08540 (US); YERRAMILLI, Kamesh R., Belle Mead, New Jersey 08502 (US)
(74) Representative: Carpmaels & Ransford LLP
(86) International application number: PCT/US2013/060918
(87) International publication number: WO 2014/052195

(56) References cited:
- EP-A2- 1 417 956
- WO-A1-95/03778
- WO-A2-2004/091553

## Description

### Field of the Invention

The compositions of this invention relate to peelable film-forming compositions that can be applied to skin for treating and removing comedones.

### Background of the Invention

Open comedones, or blackheads, are a primary sign of acne vulgaris, a disease of the hair follicles of the face, chest and back that affects a vast majority of humans during their lifetimes. A blackhead consists of a widened hair follicle filled with skin debris, bacteria and sebum and is capped with a blackened mass of skin debris. Blackheads can be unsightly and cause embarrassment to the individual who suffers with this condition.

In the past, blackhead sufferers have used skin cleansing products, including astringents, toners, surfactant-containing cleansers and the like, to cleanse and treat their skin daily. They have also utilized exfoliating implements and compositions containing exfoliating ingredients to remove dead skin and improve the tone and texture of their skin.

Blackhead treatment has been challenging in the past due to a poor understanding of blackhead formation physiology. One example of an implement used to treat and prevent blackhead formation is set forth in U.S. Patent No. 5,139,711. This patent describes a face mask that contains hydrolyzed grain end products, seaweed derivatives and water. The mask is intended to be applied to the skin, permitted to dry and then rinsed off with water, assisting in the removal of dead surface skin cells and other debris.

Other methods for exfoliation are set forth, for example, in U.S. Patent Publication No. 2004/0057921. This publication describes scrub formulations that contain polyethylene beads to help reduce the occurrence of blackheads.

Yet another method for treatment of blackheads is set forth in U.S. Patent No. 5,512,277, which describes a keratolytic plug remover composition containing a polymer with a salt forming group. This composition forms a film which can be applied to the skin, dried and then peeled off the skin.

However, it would be desirable to have a product that provides both the mechanical action to remove blackheads and to provide skin exfoliation. The mechanical action would provide immediate or fast removal of blackheads, while chemical exfoliation using an active ingredient such as salicylic acid would provide a continuous action to remove excessive dead skin cells to prevent further blackhead formation. Such a combination of mechanisms of action is not known heretofore.

### SUMMARY OF THE INVENTION

The compositions and methods of this invention relate to peelable, adhesive film-forming compositions comprising, consisting essentially of and consisting of:
(a) An anti-acne agent selected from the group consisting of: salicylic acid, sulfur and one or more alpha hydroxyacids;
(b) A high molecular weight polyvinyl alcohol component, which may be partially hydrolyzed, having the structure defined in claim 1 and having a molecular weight from 80,000 to 200,000 in the amount of from 10 to 18% by weight of the composition;
(c) An adhesion enhancing polymer component selected from the group consisting of polyesters, polyacrylates, polyvinylacrylates, polyurethanes, polyvinylcaprolactam and a combination thereof, said adhesion enhancing polymer component comprising polyvinylcaprolactam and being present in the composition in the amount of from 0.5 to 4% by weight of the composition; and
(d) A low molecular weight partially hydrolyzed polyvinylalcohol component having the structure defined in claim 1, which may be partially hydrolyzed as described herein, having a molecular weight from 10,000 to 50,000 in an amount of from 0 to 5% by weight of the composition; wherein the total weight percent of components (b) and (c) in the composition does not exceed 20%; wherein the total weight percent of components (b) and (d) in the composition does not exceed 20%; and wherein the total weight percent of components (b), (c) and (d) does not exceed 25% of the composition. Preferably, the high and low molecular weight polyvinyl alcohols useful in the compositions of this invention are hydrolyzed to a degree of from 50% to 99% more preferably from 75% to 95% and most preferably, from 87% to 89%.

### DETAILED DESCRIPTION OF THE INVENTION

The compositions of this invention contain at least one high molecular weight polyvinyl alcohol, having a structure as set forth below: 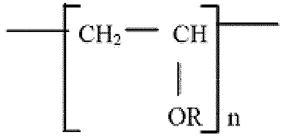 wherein R is hydrogen or -COCH_{3;} and
wherein n is from 900 to 4500 when R is hydrogen, and from 20 to 1110 when R is COCH₃. The polyvinyl alcohols useful in the compositions and methods of this invention may be partially or completely hydrolyzed.

The compositions of this invention comprise, consist essentially of and consist of at least one polyvinyl alcohol having a molecular weight from 80,000 to 200,000 and, preferably, a degree of hydrolysis from 50% to 99% more preferably from 75% to 95% and most preferably 87% to 89%, in the amount of from 10 to 18% by weight of the composition. Preferably, the high molecular weight polyvinyl alcohols are present in the compositions of this invention in an amount of from 12 to 16% by weight of the composition. More preferably, they are present in an amount of from 13 to 15% by weight of the composition.

Optionally, the compositions of this invention comprise, consist essentially of and consist of a low molecular weight polyvinyl alcohol of the following structure: 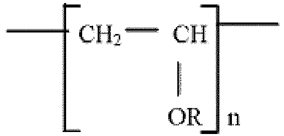 wherein R is hydrogen or -COCH_{3;} and
wherein n is from 170 to 1100 when R is hydrogen and from 2 to 300 when R is COCH₃. Such low molecular weight polyvinyl alcohols may be partially or completely hydrolyzed.

Preferably, the compositions of this invention comprise, consist essentially of and consist of at least one polyvinyl alcohol having a molecular weight from 10,000 to 50,000 and, preferably, a degree of hydrolysis from 50% to 99% more preferably from 75% to 95% and most preferably 87% to 89% in the amount of from 0 to 5% by weight of the composition. Although a second, lower molecular weight polyvinyl alcohol (i.e., 10,000 to 50,000) may be present in the compositions of this invention, it is not necessary to be present in order to achieve the superior results of the compositions of this invention.

If a low molecular weight polyvinyl alcohol is present in the compositions of this invention, the ratio between the high and low molecular weight polyvinyl alcohols should be from 2:1 to 3:1.

The compositions of this invention comprise an adhesion enhancing polymer component selected from the group consisting of polyesters, polyacrylates, polyvinylacrylates, polyurethanes, polyvinylcaprolactam and a combination thereof, wherein said adhesion enhancing polymer component comprises polyvinylcaprolactam.

The compositions of this invention contain at least one polyvinylcaprolactam, having a structure as set forth below:

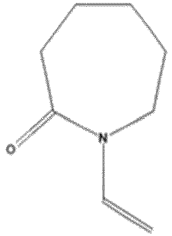

Preferably, the compositions of this invention comprise, consist essentially of and consist of at least one polyvinylcaprolactam, having a molecular weight about 150 g/mol in the amount of from 0.5 to 4% by weight of the composition.

The adhesion enhancing polymer component is present in the composition in the amount of from 0.5 to 4% by weight of the composition. More preferably, it should be present in the composition in the amount of from 0.5 to 2% by weight of the composition.

As used herein, the term "adhesive force" refers to the force required to remove from the skin a material that has been adhered, or adhesively fastened, to the skin.

As used herein, the term "film" refers to a composition that forms a thin layer or membrane on mammalian and, more particularly, human skin.

As used herein, the term "peelable" refers to materials that can be removably applied to and adhere to surfaces such as the surface of mammalian and human skin and can be subsequently removed from the skin by force. Such peelable materials can be effective to exfoliate skin and remove comedones by mechanical means. Peelable films according to the compositions and methods of this invention can be adhesively and removably applied to human skin and, by virtue of being forcibly removed, carry away the dead skin cells, hair, dirt, sebum and blackheads which have adhered to the films while leaving behind the skin benefit agents and actives onto the skin. In order to achieve this activity, the peelable films of this invention should have an adhesive force of at least 5 to 25 ounces ("oz."). The peelable films of this invention should not have such high adhesive force that they cause disruption to the outer layer of the skin and thereby damage the skin. Likewise, the adhesive force of the peelable films of this invention should be sufficiently high to enable the films to exfoliate and remove comedones from the skin.

The compositions of this invention preferably contain a film-forming polymer that functions to enhance film formation and provide some water resistance. "Film-forming polymer," as used herein, refers to polymers that when dissolved in the composition, permit a continuous or semi-continuous film to be formed when the composition is spread onto a surface such as a skin surface and the liquid vehicle is allowed to evaporate. As such, the polymer should dry on the skin surface in need of treatment such that it is spread in a predominantly continuous manner, rather than in such a way that the composition forms a plurality of discrete, island-like structures. Generally, the films formed by applying compositions on the skin according to embodiments of the invention described herein, are less than, on average, 100 microns in thickness, and preferably less than 50 microns.

The film-forming agent should preferably be present in the composition in the amount of from 1 to 10% by weight of the composition.

The compositions of this invention optionally further comprise a solvent/diluents and delivery vehicle. Upon evaporation and concentration of the solvent, the compositions of this invention can form a film that adheres to the skin. Film-forming polymers provide an additional benefit that, upon exposure to the skin and the air, the compositions of this invention form a film that can be subsequently removed, leaving behind the actives on the skin and/or simultaneously removing undesirable materials from the skin. The film can be easily removed with water or can be peeled off.

Polyvinyl alcohol compounds as set forth above act as preferred film-forming polymers in the compositions of this invention.

The compositions of this invention further comprise a skin benefit agent. A "skin benefit agent" as used herein refers to a cosmetic formulation and is not considered to be directed to the treatment of a particular condition, but possesses multifunctional properties that can contribute to the improvement of a condition. For example, glycerol (glycerin) can be included in a moisturizing cream (having a specific moisturizing agent) as an excipient, and in addition the glycerol contributes to and enhances the moisturizing properties of the cream.

One such benefit agent is a skin conditioning agent. A "conditioning agent" as used herein refers to substance that improves the quality of the skin surface, corrects or prevents skin damage. In particular, conditioning agent maintains the moisture of the skin when the film is peeled.

Examples of conditioning agents include cationic compounds with quaternary ammonium functional groups such as Polyquaternium-39 and the like.

The skin conditioning component should preferably be present in the composition in the amount of from 1 to 10% by weight of the composition, preferably from 2 to 8% by weight of the composition and more preferably from 3 to 6% by weight of the composition.

The compositions of this invention comprise an active agent. An "active ingredient" or agent used herein refers to a substance that presents specific properties used for the treatment of a particular condition. These active agents can be pharmaceutical agents, cosmetics, or wound healing agents.

Active ingredients to be delivered through the peelable film according to the invention can be any of a pharmaceutical or a cosmetic agent that are compatible with the film-forming polymers and adhesion enhancing polymers. Examples of active agent can be an antiinflammatory, an antioxidant, antimicrobial a moisturizing agent, an anti-hyperpigmentation agent, an anti-blotching agent, an anti-aging agent, an anti-collagenase substance, a free radical scavenger, a seboregulator, an hydrative, a keratolytic agent and an α-or β-hydroxy acid and the like.

Anti-acne/keratolytic agents include salicylic acid, benzoyl peroxide, sulfur, retinoic acid, alpha hydroxyacids and any of several fruit acids and the like.

The compositions of this invention optionally further comprise a color enhancing agent such as coloring agent, opacifier, and pearlizer. Such color enhancing agent include, but not limited to, zinc striate, magnesium stearate, titanium dioxide, EGMS (ethylene glycol monostearate) or Lytron 621 (Styrene/Acrylate copolymer); all of which are useful in enhancing the appearance or cosmetic properties of the skin peelable composition. A method of use of the compositions of this invention to remove undesirable materials from the skin or other surface and/or to deposit benefit agents to the surface. Preferably, the individual wishing to utilize the compositions and methods of this invention first wash his or her face with a regular, non-acne cleanser And then gently wipe with a towel until fully dry. A marble sized amount of composition according to this invention is squeezed onto the finger tip and gently applied to the desired skin surface to form a smooth, even layer. The composition is permitted to dry for 10 to 15 minutes. Once the composition is dry, the peel is gently rolled from the edge of the dried film and peeled off the skin in an upward motion.

The following are illustrative examples of the compositions of this invention. They are intended to be merely illustrative, and not limitative of the remainder of the disclosure in any way whatsoever.

### Example 1

An adhesive, peelable film-forming composition according to this invention was made by combining the ingredients set forth in Table 1 below at a temperature of 80-95°C, under pressure ambient pressure according to the following method.

For Phase A, Water was added to the beaker with propeller mixing. Subsequently, Glycerin was added to the beaker and the mixture blended until uniform. After the mixture achieved uniformity, Disodium EDTA was added and permitted to dissolve. Then, propylene glycol and olefin sulfonate-coated TiO₂ were added and the mixture blended until uniform. Once the batch was uniform, a higher molecular weight partially hydrolyzed polyvinyl alcohol was added and dispersed uniformly. Once the batch was uniformly dispersed, a lower molecular weight partially hydrolyzed polyvinyl alcohol was added to the mixture and dispersed uniformly. The batch was then heated to 85°C (the minimum cooking temperature of the partially hydrolyzed polyvinyl alcohols) and held at that temperature for 45 minutes. After 45 minutes of heating at 85°C, Polyquaternium-39, a terpolymer of acrylic acid, acrylamide and diallyldimethylammonium chloride was added to the beaker and the batch mixed until uniform. Once uniform, the mixture is cooled to below 35°C and the batch held at that temperature for phasing with Phase B.

Phase B was made by adding to a separate beaker ethyl alcohol, polyvinylcaprolactum, salicylic acid and fragrance. This blend was mixed until uniformly dissolved.

Very slowly, Phase B was added to Phase A and the resulting batch mixed until it formed a homogeneous gel.

The following compositions were made in accordance with the above-described method using the ingredients set forth in Table 1 below.

**Table 1:**

| *Formulations containing two partially hydrolyzed Polyvinyl alcohols (high molecular weight and low molecular weight) with Polyvinylcaprolactam (Example B) and without Polyvinylcaprolactam (Example A)* | | |
|---|---|---|
| **Chemical Names** | **Formula A** | **Formula B** |
| Water | 45.150 | 44.150 |
| Glycerin | 4.000 | 4.000 |
| Partially hydrolyzed Polyvinyl Alcohol (Higher Molecular Weight) | 13.500 | 13.500 |
| Partially hydrolyzed Polyvinyl Alcohol (Lower Molecular Weight) | 5.000 | 5.000 |
| Polyquaternium-39 | 1.500 | 1.500 |
| Ethyl Alcohol | 30.000 | 30.000 |
| Polyvinylcaprolactam | 0.000 | 1.000 |
| Salicylic Acid | 0.500 | 0.500 |
| TiO2, Sodium C14-16 Olefin Sulfonate | 0.200 | 0.200 |
| Disodium EDTA | 0.050 | 0.050 |
| Fragrance | 0.100 | 0.100 |
| Total % | 100.000 | 100.000 |

**Table 2:**

| *Formulations without the lower molecular weight partially hydrolyzed Polyvinyl alcohol with Polyvinylcaprolactam (Example D) and without (Example C) Polyvinyl caprolactam* | | |
|---|---|---|
| **Chemical Names** | **Formula C** | **Formula D** |
| Water | 48.75 | 47.75 |
| Glycerin | 4.000 | 4.000 |
| Propylene Glycol | 1.0 | 1.0 |
| Magnesium Aluminum Silicate | 0.4 | 0.4 |
| Partially hydrolyzed Polyvinyl Alcohol (Higher Molecular Weight) | 13.500 | 13.500 |
| Partially hydrolyzed Polyvinyl Alcohol (Lower Molecular Weight) | 0.000 | 0.000 |
| Polyquaternium-39 | 1.500 | 1.500 |
| Ethyl Alcohol | 30.000 | 30.000 |
| Polyvinylcaprolactam | 0.000 | 1.000 |
| Salicylic Acid | 0.500 | 0.500 |
| TiO2, Sodium C14-16 Olefin Sulfonate | 0.200 | 0.200 |
| Disodium EDTA | 0.050 | 0.050 |
| Fragrance | 0.100 | 0.100 |
| Total % | 100.000 | 100.000 |

### Example 2: Adhesion Measurement

A human consumer test was conducted on five healthy male and two female subjects to determine the skin adhesion of polymer films formed by a composition of blackhead removing gel Formula B vs. formula A, prepared as in Table 1. Formula A did not contain Polyvinylcaprolactam (e.g. Luviskol-plus, commercially available from BASF Corporation). Formula B contained 1% Polyvinylcaprolactam. These formulas were uniformly applied to a preselected testing site with 0.6 grams on a circle of two-inch diameter on the forearm of the subjects. After the polymer film became completely dry, a handheld scale or weight meter (30 lbs Berkley) was applied to determine the force or required to remove the film from the skin vertically. This instrument measured the adhesion force of the polymeric film attaching to the skin. Skin adhesion for two similar Formulae (Formula C and Formula D) that did not contain a low molecular weight polyvinyl alcohol was also measured. Formula C did not have polyvinylcaprolactam while Formula D had 1% polyvinylcaprolactam.

As can be seen from Table 3, addition of polyvinylcaprolactam enhanced the skin adhesion for the formula B and Formula D as compared with the corresponding control compositions, i.e.,Formula A and Formula C respectively.

**Table 3: Adhesive Force Measurement**

| Formula | Adhesive Force (oz) Average based on 7 subjects | Comments |
|---|---|---|
| Formula | 3.5+/-1 | Formula with Two PVOH's and |
| A | | No Polyvinylcaprolactum |
| Formula B | 10.5+/-1.5 | Formula with two PVOH + 1% Polyvinylcaprolactam |
| Formula C | 1.5+/-1 | Formula with one PVOH and No Polyvinylcaprolactum |
| Formula D | 8+/-0.5 | Formula with one PVOH + 1% Polyvinylcaprolactam |

### Example 3: Unexpected Effects of Blending Polyvinyl Alcohol (high molecular weight) and Polyvinylcaprolactam on the Adhesion Strength

Ten compositions, set forth in Tables 4 and 5,were made in accordance with the method set forth in Example 1. These compositions contained varying levels of high molecular weight Polyvinyl Alcohol and Polyvinylcaprolactam in the proportions set forth in the Tables 4 and 5 below and were uniformly applied to the forearm on a fixed area (0.6 grams on an area equal to the area of a circle of 2 inch diameter) on seven subjects. The polyvinyl alcohol used herein was partially hydrolyzed to the degree between 87% and 89%. After the polymer film dried completely, a handheld scale or weight meter (30 LBs Berkley) was used to determine the normal force required to remove the film from the skin. This force measures the adhesion force of the polymeric film attaching to the skin. From the results tabulated below, we observe that:

**Table 4**

| *Formulations made for studying the effect of blending polyvinyl alcohol (higher molecular weight) and Polyvinylcaprolactam on the adhesion strength* | |
|---|---|
| **Chemical Names** | **Formula** |
| Water | 48.75 |
| Glycerin | 4.000 |
| Propylene Glycol | 1.0 |
| Magnesium Aluminum Silicate | 0.4 |
| Partially hydrolyzed Polyvinyl Alcohol (Higher Molecular Weight) | See table 5 |
| Polyquaternium-39 | 1.500 |
| Ethyl Alcohol | 30.000 |
| Polyvinylcaprolactam | See table 5 |
| Salicylic Acid | 0.500 |
| TiO2, Sodium C14-16 Olefin Sulfonate | 0.200 |
| Disodium EDTA | 0.050 |
| Fragrance | 0.100 |
| Total % | 100.000 |

**Table 5 Amount of Polyvinyl Alcohol (Higher Molecular Weight) and Polyvinylcaprolactam.**

| **Formula** | **% Partially hydrolyzed Polyvinyl Alcohol (Higher Molecular weight)** | **% Polyvinyl Caprolactam** |
|---|---|---|
| 1. | 13.5 | 0 |
| 2. | 13.5 | 1 |
| 3. | 13.5 | 2 |
| 4. | 3 | 0 |
| 5. | 10 | 0 |
| 6. | 10 | 1 |
| 7. | 10 | 2 |
| 8. | 5 | 5 |
| 9. | 2 | 10 |
| 10. | 0 | 20 |

Adhesion strength of the Formulae set forth in Tables 4 and 5 was tested as set forth above. Results of these tests are recorded in Table 6 below.
a) By comparing Formulae 1, 4 and 5 (in table 6), it can be seen that in compositions that did not contain polyvinylcaprolactam, higher levels of Polyvinyl alcohol resulted in higher adhesion strength.
b) By comparing Formulae 1, 2 and 3, all of which contained 13.5% partially hydrolyzed polyvinyl alcohol, it can be observed that the adhesion strength increased in proportion to the increase in the amount of polyvinylcaprolactam present in the compositions.
This clearly indicates that the combination of partially hydrolyzed polyvinyl alcohol and polyvinylcaprolactam in accordance with the compositions of this invention provide excellent adhesion to the skin. A similar trend can be seen with respect to Formulas 5, 6 and 7 which contain 10% partially hydrolyzed polyvinyl alcohol.

**Table 6: Adhesion strength (1 oz = 28.25 g)**

| **Formula** | **% Partially hydrolyzed Polyvinyl Alcohol (Higher Molecular weight)** | **% Polyvinyl Caprolactam** | **Adhesion Strength (oz) (n = 7)** |
|---|---|---|---|
| 1. | 13.5 | 0 | 1.5+/-0.5 |
| 2. | 13.5 | 1 | 4.5+/-1.0 |
| 3. | 13.5 | 2 | 8.0 oz+/- 1.0 |
| 4. | 3 | 0 | 0+/- 0.5 |
| 5. | 10 | 0 | 0.5 +/- 0.5 |
| 6. | 10 | 1 | 3+/-1 |
| 7. | 10 | 2 | 6.5+/- 1 |
| 8. | 5 | 5 | 1.5 +/-0.5 |
| 9. | 2 | 10 | 0.5+/-0.5 |
| 10. | 0 | 20 | 0.5+/-0.5 |

## Claims

1. A peelable, adhesive film-forming composition comprising:
(a) An anti-acne agent selected from the group consisting of: salicylic acid, sulfur and one or more alpha hydroxyacids;
(b) A high molecular weight polyvinyl alcohol component having the structure 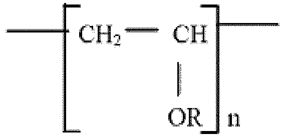
wherein R is hydrogen or -COCH_{3;} and
wherein n is from 900 to 4450 for polyvinyl alcohol R=H, and 170 to 1110 when R is COCH₃, said high molecular weight polyvinyl alcohol having a molecular weight from 80,000 to 200,000 in the amount of from 10 to 18% by weight of the composition;
(c) an adhesion enhancing polymer component selected from the group consisting of polyesters, polyacrylates, polyvinylacrylates, polyurethanes, polyvinylcaprolactam and a combination thereof, said adhesion enhancing polymer component comprising polyvinylcaprolactam and being present in the composition in the amount of from 0.5 to 4% by weight of the composition; and
(d) a low molecular weight polyvinylalcohol component having the structure 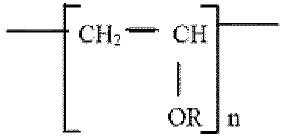
wherein R is hydrogen or -COCH_{3;} and
wherein n is from 170 to 1200 when R is hydrogen and from 2 to 290 when R is COCH₃, said low molecular weight polyvinyl alcohol having a molecular weight from 10,000 to 50,000 in an amount of from 0 to 5% by weight of the composition;
wherein the total weight percent of components (b) and (c) in the composition does not exceed 20%; wherein the total weight percent of components (b) and (d) in the composition does not exceed 20%; and wherein the total weight percent of components (b) plus (c) plus (d) does not exceed 25%.

2. A composition according to claim 1 wherein the ratio of component (b) to component (d) in the composition is from 1:1 to 6:1.

3. A composition according to claim 2 wherein the ratio of component (b) to component (d) in the composition is from 2:1 to 3:1.

4. A composition according to claim 1 wherein the anti-acne agent is salicylic acid, wherein said salicylic acid is present in the composition in an amount of from 0.5% to 2% by weight of the composition.

5. A composition according to claim 1 wherein the anti-acne agent is sulfur, wherein said sulfur is present in the composition in an amount of from 1% to 10% by weight of the composition.

6. A composition according to claim 1 wherein the anti-acne agent is one or more alpha-hydroxy acids, wherein said alpha-hydroxy acids are present in the composition in the amount of from 5% to 10% by weight of the composition.

7. A composition according to claim 1 wherein the adhesion strength of the composition is at least 85.05g (3 oz) when completely dried, as measured using a handheld scale or weight meter.

8. A composition according to claim 1 wherein the high molecular weight polyvinyl alcohol has a degree of hydrolysis from 50% to 99%.

9. A composition according to claim 1 wherein the low molecular weight polyvinyl alcohol has a degree of hydrolysis from 50% to 99%.

10. A composition according to claim 8 wherein said degree of hydrolysis is from 75% to 95%.

11. A composition according to claim 9 wherein said degree of hydrolysis is from 75% to 95%.

12. A composition according to claim 8 wherein said degree of hydrolysis is from 87% to 89%.

13. A composition according to claim 9 wherein said degree of hydrolysis is from 87% to 89%.

## Patentansprüche

1. Abziehbare, haftende, filmbildende Zusammensetzung, umfassend:
(a) ein Anti-Akne-Mittel ausgewählt aus der Gruppe bestehend aus: Salicylsäure, Schwefel und einer oder mehreren alpha-Hydroxysäuren;
(b) eine Polyvinylalkoholkomponente mit hohem Molekulargewicht mit der Struktur 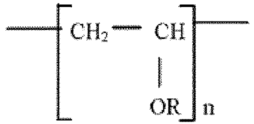 wobei R Wasserstoff oder -COCH₃ ist und
wobei n für Polyvinylalkohol mit R = H von 900 bis 4450 beträgt, und von 170 bis 1110, wenn R COCH₃ ist, wobei der Polyvinylalkohol mit hohem Molekulargewicht ein Molekulargewicht von 80.000 bis 200.000 aufweist, in einer Menge von 10 bis 18 Gew.-% der Zusammensetzung;
(c) eine haftverstärkende Polymerkomponente ausgewählt aus der Gruppe bestehend aus Polyestern, Polyacrylaten, Polyvinylacrylaten, Polyurethanen, Polyvinylcaprolactam und einer Kombination davon, wobei die haftverstärkende Polymerkomponente Polyvinylcaprolactam umfasst und in der Zusammensetzung in der Menge von 0,5 bis 4 Gew.-% der Zusammensetzung enthalten ist; und
(d) eine Polyvinylalkoholkomponente mit niedrigem Molekulargewicht mit der Struktur 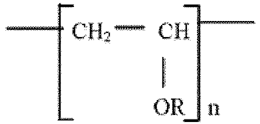 wobei R Wasserstoff oder -COCH₃ ist und
wobei n von 170 bis 1200 beträgt, wenn R Wasserstoff ist, und von 2 bis 290, wenn R COCH₃ ist, wobei der Polyvinylalkohol mit niedrigem Molekulargewicht ein Molekulargewicht von 10.000 bis 50.000 aufweist, in einer Menge von 0 bis 5 Gew.-% der Zusammensetzung;
wobei der Gesamt-Gewichtsprozentwert der Komponenten (b) und (c) in der Zusammensetzung 20 % nicht übersteigt; wobei der Gesamt-Gewichtsprozentwert der Komponenten (b) und (d) in der Zusammensetzung 20 % nicht übersteigt; und wobei der Gesamt-Gewichtsprozentwert der Komponenten (b) plus (c) plus (d) 25 % nicht übersteigt.

2. Zusammensetzung gemäß Anspruch 1, wobei das Verhältnis von Komponente (b) zu Komponente (d) in der Zusammensetzung von 1:1 bis 6:1 beträgt.

3. Zusammensetzung gemäß Anspruch 2, wobei das Verhältnis von Komponente (b) zu Komponente (d) in der Zusammensetzung von 2:1 bis 3:1 beträgt.

4. Zusammensetzung gemäß Anspruch 1, wobei das Anti-Akne-Mittel Salicylsäure ist, wobei die Salicylsäure in der Zusammensetzung in einer Menge von 0,5 Gew.-% bis 2 Gew.-% der Zusammensetzung vorhanden ist.

5. Zusammensetzung gemäß Anspruch 1, wobei das Anti-Akne-Mittel Schwefel ist, wobei der Schwefel in der Zusammensetzung in einer Menge von 1 Gew.-% bis 10 Gew.-% der Zusammensetzung vorhanden ist.

6. Zusammensetzung gemäß Anspruch 1, wobei das Anti-Akne-Mittel eine oder mehrere alpha-Hydroxysäuren ist, wobei die alpha-Hydroxysäuren in der Zusammensetzung in einer Menge von 5 Gew.-% bis 10 Gew.-% der Zusammensetzung vorhanden sind.

7. Zusammensetzung gemäß Anspruch 1, wobei die Haftstärke der Zusammensetzung, wenn vollständig getrocknet, wenigstens 85,05 g (3 oz) beträgt, wie gemessen unter Verwendung einer Handwaage oder -gewichtmessvorrichtung.

8. Zusammensetzung gemäß Anspruch 1, wobei der Polyvinylalkohol mit hohem Molekulargewicht einen Hydrolysegrad von 50 % bis 99 % aufweist.

9. Zusammensetzung gemäß Anspruch 1, wobei der Polyvinylalkohol mit niedrigem Molekulargewicht einen Hydrolysegrad von 50 % bis 99 % aufweist.

10. Zusammensetzung gemäß Anspruch 8, wobei der Hydrolysegrad von 75 % bis 95 % beträgt.

11. Zusammensetzung gemäß Anspruch 9, wobei der Hydrolysegrad von 75 % bis 95 % beträgt.

12. Zusammensetzung gemäß Anspruch 8, wobei der Hydrolysegrad von 87 % bis 89 % beträgt.

13. Zusammensetzung gemäß Anspruch 9, wobei der Hydrolysegrad von 87 % bis 89 % beträgt.

## Revendications

1. Composition filmogène adhésive pelable comprenant :
(a) un agent antiacnéique choisi dans le groupe constitué par : l'acide salicylique, le soufre et un ou plusieurs alpha-hydroxyacides ;
(b) un constituant poly(alcool vinylique) de masse moléculaire élevée ayant la structure 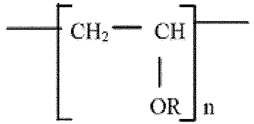 dans lequel R est l'atome d'hydrogène ou -COCH₃ ; et dans lequel n va de 900 à 4450 pour le poly(alcool vinylique) R=H et de 170 à 1110 lorsque R est COCH₃, ledit poly(alcool vinylique) de masse moléculaire élevée ayant une masse moléculaire de 80 000 à 200 000, en quantité de 10 à 18 % en poids de la composition ;
(c) un constituant polymère améliorant l'adhérence choisi dans le groupe constitué par les polyesters, les polyacrylates, les poly(acrylates de vinyle), les polyuréthanes, le polyvinylcaprolactame et une association de ceux-ci, ledit constituant polymère améliorant l'adhérence comprenant du polyvinylcaprolactame et étant présent dans la composition en quantité de 0,5 à 4 % en poids de la composition ; et
(d) un constituant poly(alcool vinylique) de faible masse moléculaire ayant la structure 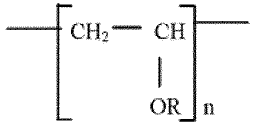 dans lequel R est l'atome d'hydrogène ou -COCH₃ ; et dans lequel n va de 170 à 1200 lorsque R est l'atome d'hydrogène et de 2 à 290 lorsque R est COCH₃, ledit poly(alcool vinylique) de faible masse moléculaire ayant une masse moléculaire de 10 000 à 50 000, en une quantité de 0 à 5 % en poids de la composition ;
le pourcentage en poids total des constituants (b) et
(c) dans la composition ne dépassant pas 20 % ; le pourcentage en poids total des constituants (b) et (d) dans la composition ne dépassant pas 20 % ; et le pourcentage en poids total des constituants (b) plus (c) plus (d) ne dépassant pas 25 %.

2. Composition selon la revendication 1, le rapport du constituant (b) au constituant (d) dans la composition étant de 1:1 à 6:1.

3. Composition selon la revendication 2, le rapport du constituant (b) au constituant (d) dans la composition étant de 2:1 à 3:1.

4. Composition selon la revendication 1 dans laquelle l'agent anti-acné est l'acide salicylique, ledit acide salicylique étant présent dans la composition en une quantité de 0,5 % à 2 % en poids de la composition.

5. Composition selon la revendication 1 dans laquelle l'agent anti-acné est le soufre, ledit soufre étant présent dans la composition en une quantité de 1 % à 10 % en poids de la composition.

6. Composition selon la revendication 1 dans laquelle l'agent anti-acné est un ou plusieurs alpha-hydroxyacides, lesdits alpha-hydroxyacides étant présents dans la composition en quantité de 5 % à 10 % en poids de la composition.

7. Composition selon la revendication 1, la force d'adhérence de la composition étant d'au moins 85,05 g (3 oz) lorsqu'elle est complètement séchée, telle que mesurée à l'aide d'une balance portative ou d'un dispositif de mesure de poids portatif.

8. Composition selon la revendication 1 dans laquelle le poly(alcool vinylique) de masse moléculaire élevée a un degré d'hydrolyse de 50 % à 99 %.

9. Composition selon la revendication 1 dans laquelle le poly(alcool vinylique) de faible masse moléculaire a un degré d'hydrolyse de 50 % à 99 %.

10. Composition selon la revendication 8 dans laquelle ledit degré d'hydrolyse est de 75 % à 95 %.

11. Composition selon la revendication 9 dans laquelle ledit degré d'hydrolyse est de 75 % à 95 %.

12. Composition selon la revendication 8 dans laquelle ledit degré d'hydrolyse est de 87 % à 89 %.

13. Composition selon la revendication 9 dans laquelle ledit degré d'hydrolyse est de 87 % à 89 %.
